(19) Europäisches Patentamt
European Patent Office
Office européen des brevets

(11) Numéro de publication : **0 458 886 B1**

(12) **FASCICULE DE BREVET EUROPEEN**

(45) Date de publication du fascicule du brevet : **13.10.93 Bulletin 93/41**

(51) Int. Cl.$^5$ : **A61K 31/165,** A61K 31/18, A61K 31/63, C07C 233/78, C07C 311/18

(21) Numéro de dépôt : **90903857.2**

(22) Date de dépôt : **14.02.90**

(86) Numéro de dépôt international : **PCT/FR90/00107**

(87) Numéro de publication internationale : **WO 90/09170 23.08.90 Gazette 90/20**

# (54) AGENTS POUR LE DIAGNOSTIC ET LE TRAITEMENT DES MELANOMES, DERIVES HALOGENES AROMATIQUES UTILISABLES COMME DE TELS AGENTS ET LEUR PREPARATION.

(30) Priorité : **14.02.89 FR 8901898**

(43) Date de publication de la demande : **04.12.91 Bulletin 91/49**

(45) Mention de la délivrance du brevet : **13.10.93 Bulletin 93/41**

(84) Etats contractants désignés : **AT BE CH DE DK ES FR GB IT LI LU NL SE**

(56) Documents cités :
**DE-A- 3 407 654**
**Eur. J. Med. Chem. - Chim. Ther., vol. 21, no. 5, 1986 (Paris, FR), M.-F. Moreau et al., pp. 423-431**
**Pharmacol. Res. Communic., vol. 20, suppl. IV, 1988, The Italian Pharmacol. Soc., A.M. Cesura et al., pp. 51-61**
**Advances in Neurology, vol. 45, 1987, Parkinson's Dis., New York, US), M. Da Prada et al., pp. 175-178**

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**
Titulaire : **CIS BIO INTERNATIONAL**
**RN 306**
**F-91000 Saclay (FR)**

(72) Inventeur : **MOREAU, Marie-France**
**Impasse Voltaire**
**F-63540 Romagnat (FR)**
Inventeur : **MICHELOT, Josette**
**17 bis, route de Chanonat La Roche-Blanche**
**F-63670 Le Cendre (FR)**
Inventeur : **VEYRE, Annie**
**50, avenue Julien**
**F-63000 Clermont-Ferrand (FR)**
Inventeur : **MADELMONT, Jean-Claude**
**32, boulevard du Chauffour**
**F-63540 Romagnat (FR)**
Inventeur : **GODENECHE, Denise**
**84, boulevard Thermal**
**F-63400 Chamalières (FR)**
Inventeur : **LABARRE, Pierre**
**142, avenue Léon-Blum**
**F-63000 Clermont-Ferrand (FR)**
Inventeur : **PARRY, Daniel**
**11 bis, rue de Ceyrat**
**F-63110 Beaumont (FR)**
Inventeur : **MEYNIEL, Gaston**
**77, avenue Raymond-Bergougnan**
**F-63000 Clermont-Ferrand (FR)**

(74) Mandataire : **Polus, Camille et al**
**c/o Cabinet Lavoix 2, Place d'Estienne d'Orves**
**F-75441 Paris Cedex 09 (FR)**

EP 0 458 886 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des agents pour le diagnostic et le traitement des mélanomes ma- lins et de nouveaux dérivés halogénés aromatiques éventuellement marqués utilisables comme de tels agents.

Le mélanome malin est l'une des plus dangereuses tumeurs de la peau avec une incidence en augmentation régulière. La survie à 5 ans ne dépasse pas 14% sauf dans le cas où l'épaisseur de la tumeur est inférieure à 0,76 mm. Dans le cas où la lésion dépasse cette épaisseur, cette tumeur donne des métastases de façon imprévisible et silencieuse. C'est pourquoi on recherche actuellement une méthode d'investigation qui permette d'évaluer précocement aussi bien l'extension locale que l'extension à distance de la tumeur.

Durant ces dix dernières années, un certain nombre de produits radiopharmaceutiques, retenus pour leur potentielle affinité pour la mélanine, ont été expérimentés mais peu ont eu un développement clinique satisfaisant. Nous ne citerons que les iodoquinolines, iodométhyltyrosines aujourd'hui abandonnées et le citrate de gallium 67 dont les inconvénients sont bien connus : élimination très lente à partir du tractus digestif, localisation tardive et importante dans le foie et le rein pouvant rendre difficile la visualisation de la tumeur. Par ailleurs le marquage se fait de façon non spécifique sur les foyers inflammatoires, infectieux ou le tissu tumoral en voie de développement.

De nombreuses recherches s'attachent à montrer l'utilité des anticorps monoclonaux pour l'immunoscintigraphie des tumeurs. Les premiers essais ont été réalisés avec un anticorps monoclonal marqué $^{131}I$ dirigé contre la protéine p 97 présente dans la majorité des cellules de mélanome humain. Les expérimen- tations chez l'animal, puis l'étude en clinique, ont montré la supériorité de l'utilisation d'un anticorps dirigé contre un fragment de la protéine p 97, F (a b')2 par rapport à l'anticorps dirigé contre la protéine complète, pour la réalisation des immunoscintigraphies. Ces anticorps permettent de détecter des mélanomes de taille supérieure à 1,5 cm mais la sensibilité est influencée par le site anatomique des tumeurs et par leur teneur en antigènes. La limite majeure de l'immunoscintigraphie reste l'accumulation non spécifique de la radio-activité dans la moëlle osseuse, la rate, le foie, le rein. Il paraît impératif d'améliorer soit le marquage et/ou la purification de l'anticorps pour diminuer les activités rénales et hépatiques.

Plus récemment l'iodothiouracile marqué par $^{125}I$ a été étudié chez des animaux porteurs de tumeurs greffées. Il semble être un agent prometteur pour la localisation et le traitement du mélanome malin : cependant le marquage de ce composé par synthèse totale n'est pas encore adapté à l'utilisation de $^{123}I$. Par ailleurs son incorporation comme pseudo-précurseur de la mélanine au cours du processus de biosynthèse peut en limiter l'emploi. Des études préliminaires menées avec des porphyrines marqués par $^{111}In$ ont permis de détecter des tumeurs greffées chez l'animal. Enfin $^{123}I$-IMP a été utilisé pour la détection du mélanome chez l'homme. Une étude comparative avec les anticorps monoclonaux antimélanomes marquis par $^{111}In$ a montré une sensibilité supérieure de l'immunoscintigraphie.

D'un autre côté les présents auteurs ont décrit des composés benzéniques marqués par $^{125}I$, utilisables en médecine nucléaire pour l'exploration du système nerveux central (Eur. J. Med. Chem.-Chim. Ther. 1986, 21, N°5, 423-31). Dans le même sens, voir Adv. Neurol., Vol. 45, 1987, pages 175-178.

On a maintenant trouvé que ces composés ainsi que de nouveaux composés de structure analogue peuvent être utilisés comme traceurs de mélanomes.

La présente invention a donc pour objet l'utilisation, pour la préparation d'un agent pour le diagnostic et le traitement des mélanomes malins, des substances répondant à la formule générale

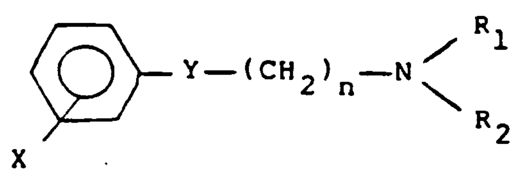

(I)

dans laquelle

X représente un atome d'hydrogène ou d'halogène,

Y représente un groupement Z-CONH-,Z-O-,Z-S(O)-$_{n1}$ avec $n_1$ = 0, 1,2 ou 3, Z-$SO_2$-NH- ou Z-NHCO-, Z étant une liaison, $CH_2$- ou

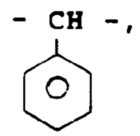

n est un entier de 2 à 4, et

$R_1$ et $R_2$ représentent chacun un atome d'hydrogène, un groupe alkyle en $C_1$ à $C_6$, aryle ou aralkyle en $C_6$ à $C_{12}$,

ces composés pouvant être marqués par des radioisotopes choisis parmi $^{123}I$ $^{125}I$, $^{131}I$ $^{75}Br$, $^{77}Br$, $^{18}F$, ou $^{11}C$, ou des isotopes stables choisis parmi $^{13}C$ et $^{19}F$, ainsi que leurs sels d'addition à des acides pharmaceutiquement aceptables. Certains des produits précédents ont déjà reçu une utilisation dans le domaine thérapeutique, par exemple comme anti-dépresseurs et anti-parkinsoniens (DE-A-34 07 654).

L'invention concerne plus particulièrement l'utilisation des agents radiopharmaceutiques de formule(I) dans laquelle X représente $^{123}I$, $^{125}I$, ou $^{131}I$, Y représente Z-CONH- ou Z-$SO_2$NH-, Z étant une liaison, -$CH_2$- ou -

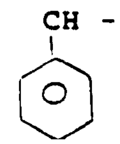

et

$R_1$ et $R_2$ représentent chacun un atome d'hydrogène ou un groupe alkyle en $C_1$ à $C_6$.

Les agents pharmaceutiques répondant à la formule générale

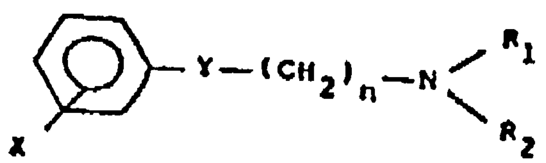 (I)

dans laquelle

X représente un atome d'hydrogène ou d'halogène,

Y représente un groupement Z-CONH-, Z-O-, Z-S(O)-$_{n1}$ avec $n_1$ = 0, 1, 2 ou 3, Z-$SO_2$-NH- ou Z-NHCO-, Z étant une liaison, -$CH_2$- ou

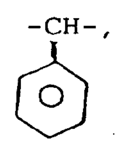

n est un entier de 2 à 4, et

$R_1$ et $R_2$ représentent chacun un atome d'hydrogène, un groupe alkyle en $C_1$ à $C_6$, aryle ou aralkyle en $C_6$ à $C_{12}$, ces composés pouvant être marqués par des radioisotopes choisis parmi $^{123}I$, $^{125}I$, $^{131}I$, $^{75}Br$, $^{77}Br$, $^{18}F$, ou $^{11}C$, ou des isotopes stables choisis parmi $^{13}C$ et $^{19}F$,

ainsi que leurs sels d'addition à des acides pharmaceutiquement acceptables, à l'exclusion des composés de formule (I), dans laquelle Y représente Z-CONH-, Z étant une liaison, n représente 2, et $R_1$ et $R_2$ représentent simultanément un atome d'hydrogène, des composés de formule (I), dans laquelle Y représente Z-CONH-, Z étant une liaison, et X représente F, Cl, Br en position ortho, méta ou para ou I en position ortho ou méta, des composés de formule (I)dans laquelle Y représente Z-$SO_2$-NH, Z étant une liaison, et X représente Cl en position para et du composé de formule (I) dans laquelle Y représente Z-CO-NH-, Z étant une liaison, X est un atome d'iode en position para, et $R_1$ et $R_2$ représentent un groupe éthyle, constituent également un autre objet de l'invention.

Les agents précédents, répondant à la formule (I) dans laquelle X représente $^{123}I$, $^{125}I$ ou $^{131}I$, Y répresente

Z-CONH- ou Z-$SO_2$-NH-, Z étant une liaison, -$CH_2$- ou

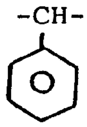

et

$R_1$ et $R_2$ représentent chacun un atome d'hydrogène, ou un groupe alkyle en $C_1$ à $C_6$ sont particulièrement préférés.

Parmi ces agents (I), les composés de formule générale (II) suivantes sont également distingués:

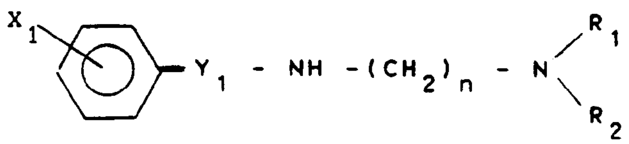

(II)

dans laquelle
$X_1$ représente un atome d'halogène ou un de ses radioisotopes,
$Y_1$ représente un groupe CO ou $SO_2$,
n est un entier de 2 à 4, et
$R_1$ et $R_2$ représentent chacun un atome d'hydrogène ou un groupe alkyle en $C_1$ à $C_6$,
ainsi que leurs sels d'addition à des acides pharmaceutiquement acceptables, à l'exclusion des composés de formule (II) dans laquelle $Y_1$ représente CO, n représente 2, et $R_1$ et $R_2$ représentent simultanément l'hydrogène, des composés de formule générale (II), dans laquelle $Y_1$ représente CO, $X_1$ représente F, Cl, Br en position ortho, méta ou para ou I en position ortho ou méta, des composés de formule (II) dans laquelle $Y_1$ représente $SO_2$ et $X_1$ représente Cl en position para et du composé de formule (II) dans laquelle $Y_1$ représente CO, $X_1$ est un atome d'iode en position para et $R_1$ et $R_2$ représentent un groupe éthyle.

Les composés de formule (II) peuvent être préparés par condensation d'un chlorure d'acide halogénobenzoïque ou -benzènesulfonique de formule

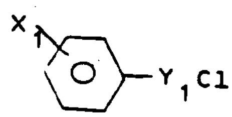

(III)

dans laquelle $X_1$ et $Y_1$ ont la même signification que dans la formule (II), avec une diamine de formule

$$NH_2\text{-}(CH_2)_n\text{-}NR_1R_2 \qquad (IV)$$

dans laquelle n, $R_1$ et $R_2$ ont la même signification que dans la formule II, puis marquage éventuel de la structure (II) ainsi obtenue par un radioisotope de l'halogène $X_1$, notamment $^{123}I$, $^{125}I$, $^{131}I$, $^{75}Br$, $^{77}Br$ ou $^{18}F$.

La réaction de condensation entre (III) et (IV) peut être ,effectuée à température ambiante, dans un solvant neutre tel que le tétrahydrofuranne (THF). La réaction est effectuée de préférence avec un excès de diamine (IV), en particulier d'environ 2 moles de (IV) pour une mole de (III).

Les composés de formule (II) dans laquelle $Y_1$ = CO peuvent également être préparés par condensation d'un chlorure d'acide halobenzoïque (III) avec le paranitrophénol pour obtenir un ester de formule

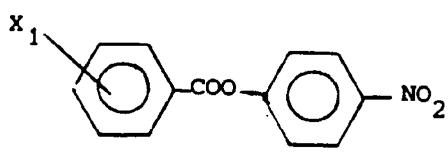

V

puis réaction du composé (V) avec une diamine (IV), et enfin marquage éventuel de la structure (II) ainsi obtenue par un radioisotope de $X_1$.

La réaction de condensation de l'acide (III) avec le paranitrophénol peut être effectuée à une température de 30° à 50°C, dans un solvant neutre tel que le THF, et en présence d'un accepteur d'acide (carbonate, amine).

La réaction entre les composés (V) et (IV) peut être effectuée à température ambiante dans un solvant neutre tel que le THF, de préférence avec un excès de diamine (IV), en particulier d'environ 2 moles de (IV) pour une mole de (V).

Le marquage de la structure (II) est effectuée par échange en milieu acide entre la molécule iodée non radioactive et un halogénure alcalin radioactif, par exemple Na $^{125}I$. L'échange peut être réalisé par chauffage au reflux d'une solution aqueuse du composé (II) et de l'halogénure radioactif, acidifiée par exemple par l'acide acétique en présénce de sulfate de cuivre.

Les exemples suivants illustrent l'invention.

EXEMPLE 1 :

N-(diéthylamino-2-éthyl) iodo-4 benzamide et son chlorhydrate (produit A).

a) On laisse réagir une nuit à température ambiante une solution de 20 mM de N,N-diéthyl éthylènediamine du commerce avec 10 mM de chlorure d'acide paraiodobenzoïque (préparé à partir de 10 mM d'acide paraiodobenzoïque et de 10 mM de chlorure de thionyle) dans 50 ml de THF. On évapore le solvant, reprend par l'eau et extrait au chloroforme. On sèche sur $MgSO_4$, puis on purifie sur colonne de chromatographie (silice; éluant :gradient $CHCl_3$-EtOH de 5% à 20% ou $CHCl_3$-EtOH-$Et_2NH$ 92% - 6% - 2% v/v). Rendement : environ 80%.

Préparation du chlorhydrate

Le composé A, isolé sous forme base, est solubilisé dans le minimum de chloroforme, et traité par HCl/éther (≃ 2N) jusqu'à précipitation totale. On évapore sous vide et lave le chlorhydrate formé à l'éther anhydre, et filtre. F 184-185°C.

IR (pastille KBr ,ν $cm^{-1}$) : 3220 (NH); 3030 (CH aromatique); 1970 ($CH_3$); 2930 ($CH_2$); 1635 (CO); 1575,1520 (C-NH).

$^1H$-RMN (solvant : méthanol (D4) : 1,35 (t, 6H, $C\underline{H}_3$; 3,10-3,53 (m, 6H, $C\underline{H}_2$-N-($C\underline{H}_2CH_3)_2$-); 3,65-3,90 (m, 2H, CONH-$C\underline{H}_2$); 7,46-7,93 (m, 4H, aromatiques).

b) marquage par $^{125}I$

Méthode 1 : Produit A (52 μM) en solution dans 1 ml d'eau. Acide acétique : 250 ul. Sulfate de cuivre : 80 μM. Na $^{125}I$. 45 mn à 170°. Hydrolyse (NaOH N). Passage sur "Extrelut" (élution chloroforme). Eva-poration. Traitement du résidu par HCl, éther. Le produit A est isolé sous forme chlorhydrate. Rendement radiochimique : 95%. Pureté radiochimique >98% (CCM, HPLC).

Méthode 2 : Produit A (26 μM) en solution dans 500 μl de tampon acétate 0,1 M, pH 4,62. Sulfate de cuivre : 1,26 μM. Na $^{125}I$. 45 mn à 120°. CCM, HPLC du milieu -- pureté radiochimique >97,5% (perte de radioactivité observée <7%).

Méthode 3 : Produit A (26 μM) en solution dans 500 μl de tampon citrate 0,05 M, pH 4. Sulfate de cuivre : 3 μM. Na $^{125}I$. 35 mn à 150°. CCM, HPLC du milieu -- pureté radiochimique >98% (perte de radioactivité observée <3%).

EXEMPLE 2 :

N-(éthylamino-2 éthyl) iodo-4 benzamide et son chlorhydrate (produit B).

a) p-iodobenzoate de p-nitrophényle

On fait réagir une nuit à 40°C 2 mM de p-nitrophénol avec 2 mM de chlorure de l'acide p-iodobenzoïque, en présence 2 mM de triéthylamine dans 20 ml de THF. On évapore le solvant et reprend le résidu par le chloroforme et lave avec HCl1N, puis $H_2O$. On sèche sur $MgSO_4$. On purifie sur colonne de chromatographie (silice; éluant $CHCl_3$-hexane 85/15 v/v). Rendement : 90%.

b) on fait réagir l'ester précédent (1 mM) avec de la N-éthyléthylènediamine du commerce; 2 mM) dans 20 ml de THF pendant 24 h. à température ambiante. On évapore le solvant, reprend par l'eau et extrait au chloroforme. On lave la phase organique avec NaOH1N, puis $H_2O$. On sèche sur $MgSO_4$. On purifie sur colonne de chromatographie (silice; éluant $CHCl_3$-EtOH-$Et_2NH$ 92% 6% 2% v/v). On prépare le chlorhydrate du composé du titre de la même manière que dans l'exemple 1. F = 242-243°C.

IR (pastille KBr, $cm^{-1}$) : 3280 (NH); 1635 (CO); 1575 (C-NH); 1540 (NH).

$^1H$-RMN (solvant : DMSO $D_6$ + $D_2O$ + $CF_3$ COOH) : 1,20 (t, 3H, $C\underline{H}_3$); 2,83-3,27 (m, 4H, -$C\underline{H}_2$-NH-$C\underline{H}_2$-$CH_3$); 3,43-3,70 (m, 2H, CONH-$C\underline{H}_2$); 7,50-7,93 (m, 4H, aromatiques).

c) marquage par $^{125}I$

Méthode 1 : Le protocole utilisé est le même que celui décrit pour le composé A dans l'exemple 1.

Rendement radiochimique : 90%. Pureté radiochimique >98% (CCM,HPLC).

Méthode 2 : Même protocole que dans l'exemple 1. Pureté radiochimique 97% (CCM).

Ces composés ainsi que d'autres dérivés de formule II préparés de manière analogue sont rassemblés dans le tableau suivant :

**TABLEAU**

| Dérivé | $X_1$ | $Y_1$ | n | $R_1$ | $R_2$ |
|---|---|---|---|---|---|
| A(*) | para-I | CO | 2 | $C_2H_5$ | $C_2H_5$ |
| B | para-I | CO | 2 | H | $C_2H_5$ |
| C | ortho-I | CO | 2 | $C_2H_5$ | $C_2H_5$ |
| D | para-I | CO | 2 | H | H |
| E | para-I | CO | 2 | $CH_3$ | $CH_3$ |
| F | para-I | CO | 2 | H | $CH_3$ |
| G | para-I | CO | 2 | $CH(CH_3)_2$ | $CH(CH_3)_2$ |
| H | para-I | CO | 2 | H | $CH(CH_3)_2$ |
| J | para-I | CO | 3 | $CH_3$ | $CH_3$ |
| K | para-I | CO | 3 | H | $CH_3$ |
| L | para-I | CO | 3 | $C_2H_5$ | $C_2H_5$ |
| M | para-I | $SO_2$ | 2 | $C_2H_5$ | $C_2H_5$ |

(*)décrit dans Eur. J. Med. Chem. précité sous le N°14

Les points de fusion sont pris sur Banc Kofler.

Les spectres $^1H$-RMN sont effectués sur un appareil JEOL C60 HL, les déplacements chimiques sont exprimés en ppm par rapport au TMS utilisé comme référence interne.

**Produit C :**

$F_{(°C)}$ = 169-170

$^1H$-RMN (solvant : Méthanol $D_4$) : 1,40 (t, 6H, $C\underline{H}_3$) ; 3,0-4,0 (m, 8H, CONH $C\underline{H}_2C\underline{H}_2$-N($C\underline{H}_2$ $CH_3)_2$) ; 7,07-7,63 (m, 3H, aromatiques) ; 7,87-8,10 (m, 1H, aromatique).

**Produit D :**

$F_{(°C)}$ > 250

$^1H$-RMN (solvant : Méthanol $D_4$) ; 3,03-3,43 (m, 2H, $C\underline{H}_2NH_2$) ; 3,53-3,83 (m, 2H, CONH $C\underline{H}_2$) ; 7,50-7,93 (m, 4H, aromatiques).

**Produit E :**

$F_{(°C)}$ = 240-241

$^1H$-RMN (solvant : méthanol $D_4$) : 3,0 (s, 6H, $C\underline{H}_3$) ; 3,27-3,60 (m, 2H, $C\underline{H}_2$ $N(CH_3)_2$) ; 3,67-4,0 (m, 2H, CONH $C\underline{H}_2$) ; 7,53-8,07 (m, 4H, aromatiques)

**Produit F :**

$F_{(°C)}$ >250

$^1$H-RMN (solvant : DMSO + $D_2O$) : 2,67 (s, 3H, $C\underline{H}_3$) ; 3,03-3,33 (m, 2H, $C\underline{H}_2$-NH-$CH_3$) ; 3,50-3,77 (m, 2H, CONH $C\underline{H}_2$) ; 7,53-8,0 (m, 4H, aromatiques)

**Produit G :**

$F_{(°C)}$= 185-186
$^1$H-RMN (solvant : méthanol $D_4$) : 1,37 et 1,47 (2s, 12 H, $C\underline{H}_3$) ; 3,23-4,0 (m, 6H, $C\underline{H}_2$, et $C\underline{H}$) ; 7,57-8,0 (m, 4H, aromatiques)
$F_{(°C)}$ = 219-220
$^1$H-RMN (solvant : méthanol $D_4$) : 1,30 et 1,43 (2s, 6H, $C\underline{H}_3$) ; 3,13-3,50 (m, 3H, $C\underline{H}_2$ NH-$C\underline{H}$ $(CH_3)_2$) ; 3,57-3,90 (m, 2H, CONH $C\underline{H}_2$) ; 7,50-7,93 (m, 4H, aromatiques)

**Produit J :**

$F_{(°C)}$ = 165-166
$^1$H-RMN (solvant : méthanol $D_4$) : 1,77-2,33 (m, 2H, $CH_2$-$C\underline{H}_2$-$CH_2$) ; 2,93 (s, 6H, $C\underline{H}_3$) ; 3,0-3,67 (m, 4H, CONH $C\underline{H}_2$-$CH_2C\underline{H}_2$); 7,47-7,97 (m, 4H, aromatiques)

**Produit K :**

$F_{(°C)}$ = 243-244
$^1$H-RMN (solvant : méthanol $D_4$) : 1,87-2,30 (m, 2H, $CH_2C\underline{H}_2CH_2$) ; 2,73 (s, 3H, $C\underline{H}_3$) ; 2,93-3,23 (m, 2H, $C\underline{H}_2$ NH $CH_3$) ; 3,33-3,70 (m, 2H, CONH $C\underline{H}_2$) ; 7,50-8,0 (m, 4H, aromatiques)

**Produit L :**

$F_{(°C)}$ = 88-90
$^1$H-RMN (solvant : méthanol $D_4$) : 1,33 (t, 6H, $C\underline{H}_3$) ; 1,93-2,40 (m, 2H, CONHCH$_2$ $C\underline{H}_2$ $CH_2$) ; 3,03-3,70 (m, 8H, CONH $C\underline{H}_2CH_2C\underline{H}_2$ N($C\underline{H}_2CH_3)_2$) ; 7,53-7,97 (m, 4H, aromatiques)

**Produit M :**

$F_{(°C)}$ = 154-155
$^1$H-RMN (solvant : méthanol $D_4$) : 1,33 (t, 6H, $C\underline{H}_3$) ; 3,0-3,53 (m, 8H, $C\underline{H}_2$) ; 7,50-8,13 (m, 4H, aromatiques)

Les produits de l'invention ont été soumis à des essais pharmacologiques et biologiques.

1. Etude de toxicité

La détermination de la DL50 est faite chez la souris après injection i.v. des molécules d'après la méthodologie décrite par Karber et Behrens.

Elle a été réalisée chez :
- des souris mâles OF 1, de 6 semaines
- des souris nudes nu/nu SSCUP, mâles âgées de 6 semaines
- des souris nudes nu/nu SSCUP, mâles porteuses d'hétérotransplants de mélanomes humains.

a. après l'injection i.v. du dérivé A.

Souris mâles OF 1 de 6 semaines.

| | |
|---|---|
| DLO : | 75 mg/kg |
| DL50 : | 125 mg/kg |
| DL 100 : | 150 mg/kg |

**Souris nudes nu/nu SSCUP, mâles, 6 semaines.**

**DL 0 : 60 mg/kg**

**DL 50 : 90 mg/kg**

**DL 100 : 150 mg/kg**

**Souris nudes nu/nu SSCUP, mâles, porteuses d'hétérotransplants (mélanome Beu)**

**DL 0 : 60 mg/kg**

**DL 50 : 90 mg/kg**

**DL 100 : 150 mg/kg**

b. après l'injection i.v. dérivé B.

**Souris mâles OF 1,6 semaines.**

DL 0 : 150 mg/kg

DL 50 : 175 mg/kg

DL 100 : 200 mg/kg

2. Autoradiographies

Les autoradiographies sont réalisées chez des souris porteuses de mélanomes greffés :

a. C57 BL6 porteuses du mélanome murin B16.

b. nudes nu/nu SSCUP porteuses d'hétérotransplants de mélanomes humains mélaniques (Beu) ou amélaniques (Dau). L'intérêt de ce modèle réside dans le fait que ces tumeurs humaines gardent leurs propriétés histologiques et biochimiques.

Les animaux porteurs de tumeurs greffées reçoivent dans une veine caudale les composés marqués sous forme de chlorhydrate en solution saline (3.7 à $5.10^5$ B g/animal). A des intervalles de temps différents après l'administration des produits (de 15 minutes à 24 heures), les souris sont anesthésiées à l'éther et plongées dans l'azote liquide.

Selon le protocole décrit par Ullberg des coupes de 30 μm, parallèles au plan sagittal, sont réalisées dans un cryotome à congélation (TS 260, SLEE). Les coupes déshydratées au froid sont appliquées sur des films sensibles, Ultrofim $^3H$ (LKB, 2208-190) ou Hyperfilm $^3H$ (Amerham, RPN 535). Après 3 semaines d'exposition les films sont révélés selon les méthodes classiques de développement.

c. Résultats.

De 1 à 4 h après l'injection i.v. de $^{125}I$-A chez des souris C57 BL6 porteuses du mélanome B16, on peut noter un noircissement important du film au niveau de la tumeur, noircissement non homogène. Cette hétérogénéité correspond à des zones de nécrose de la tumeur.

Des images identiques, aux mêmes temps, sont obtenues chez des souris nudes nu/nu SSCUP porteuses d'hétérotransplants de mélanome humain mélanique, Beu. 24 h après l'injection le mélanome B16 est encore visible mais nous ne notons plus de noircissement du film au niveau de la tumeur Beu.

1h après l'injection de $^{125}I$-B, chez des souris porteuses du mélanome B16, le noircissement du film au niveau de la tumeur est qualitativement aussi important qu'après l'injection de A. La cinétique est légèrement différente avec une concentration radioactive tumorale plus intense à 4 h et surtout 6 h. 24 heures après l'injection la tumeur est encore visible alors qu'il ne reste plus de radioactivité dans l'organisme.

L'hétérotransplant Beu est visible jusqu'à 6 h après l'injection, et, à ce temps présente un noircissement

plus prononcé sur le pourtour de la tumeur.

Après l'injection i.v. de cellules de mélanome B16 on note l'apparition de colonies métastasiantes en majorité pulmonaires. Ces colonies pulmonaires sont bien visualisées sur les autoradiographies de 1 à 7 heures après l'injection de B.

3. Distribution tissulaire chez l'animal

L'étude de la distribution tissulaire des composés marqués $^{125}I$ a été réalisée chez des souris porteuses de mélanomes greffés (cf. autoradiographies ci-dessus).

Les animaux reçoivent une injection dans une veine caudale de 3 µmoles, 13,5 à $29{,}6.10^4$ Bg de produit marqué sous forme de chlorhydrate. Les animaux sont sacrifiés à des intervalles de temps différents après l'injection, de 1 à 24 heures. Le sang est prélevé et les principaux organes sont excisés. Des aliquotes sont pesées et la radioactivité des échantillons est mesurée dans un compteur Gammatrac 1191 (Traceur analytique). Le rendement de comptage pour l'iode 125 est de 60%.

Souris porteuses de mélanomes.

Les résultats sont exprimés :

- en pourcentage de la dose injectée fixé par g de tissu (% DI/g);
- sous forme des rapports $\frac{\text{radioactivité tissu}}{\text{radioactivité sang}}$

Ce rapport reflète plus précisément la sélectivité d'une molécule pour un organe (tableaux 2, 4, 6, 8, 10 et 12).

Dérivé A

Jusqu'à 8 heures après l'injection de A à des souris porteuses de mélanomes mélaniques, B16 et Beu, on note des concentrations intratumorales supérieures à 1% DI/g avec des valeurs toujours plus élevées pour B16. Entre 6 et 12 heures les rapport tu- meur/sang sont de l'ordre de 20 pour les 2 tumeurs. 24 h après l'injection si le rapport pour B16 est supérieur à 30, celui de Beu n'est que de 6. (cf. tableaux 1 à 4 ci-après).

La fixation pulmonaire relativement importante pendant les 4 premières heures devient inférieure à 1% DI/g dès la 6ème heure.

Cette étude permet aussi de constater que 6 heures après l'injection les mélanomes mélaniques sont les tissus qui présentent les pourcentages de fixation et les rapports tissu/sang les plus élevés. Les rapports tumeur/muscle, poumon, cerveau et foie, tous très supérieurs à l'unité de 6 à 24 heures après l'injection chez les souris porteuses de Beu ou B16 sont des éléments positifs pour une étude scintigraphique de recherche de métastases.

Pour le mélanome amélanique (Dau) la concentration intratumorale de A n'est supérieure à 1% DI/g que pendant les 2 heures qui suivent l'injection. Les rapports tumeur/sang qui varient entre 1 et 2 de 1 à 24 heures ont cependant permis d'obtenir des images (cf. tableaux 5 et 6 ci-après).

Dérivé B

Les résultats préliminaires montrent pour les mélanomes mélaniques des concentrations intratumorales et des rapports nettement supérieurs à ceux observés avec A de 4 à 8 h après l'injection. Par contre au temps 24 heures les concentrations et les rapports tumeur/sang sont inférieurs à ceux de A (tableaux 7, 8 ci-après).

Par ailleurs si l'on cherche à déterminer un "index" de contraste des tumeurs mélaniques par rapport aux muscles avoisinants ou aux organes susceptibles d'être le siège de métastases on peut constater qu'au temps 6 heures la majorité de ces index est plus élevée pour B que pour A, résultat en faveur de l'utilisation de B pour l'imagerie à ce temps.

En ce qui concerne l'analyse des résultats chez les animaux porteurs de la tumeur amélanique, les concentrations intratumorales sont légèrement plus élevées de 4 à 6 h mais les rapports tumeur/sang ne sont pas significativement différents (tableaux 9 et 10 ci-après).

On a en outre étudié les dérivés E, G, J et L. Tous sont comme les dérivés A et B des marqueurs du mélanome murin B16.

Le dérivé G présente une cinétique d'élimination proche de celle du dérivé A, tandis que les dérivés E, J et L présentent une élimination plus rapide. Le dérivé E laisse entrevoir des possibilités de scintigraphie entre 3h et 6h après son administration.

4. Imagerie

Les souris porteuses de tumeurs greffées sont anesthésiées par injection I P de pentobarbital sodique 1/5 et immobilisées sous une caméra à scintillation (Gammatome II, CGR) munie d'un collimateur pinhole 3-360, sensibilité 3,2 $10^{-4}$. Les images ont été obtenues après l'injection i.v. de 60 à 100 uCi de $^{125}I$ et $^{123}I$-A, chez des souris :

- C57 BL6 porteuses de mélanomes B16 à différents stades d'évolution.
- C57 BL6 porteuses de métastases pulmonaires de mélanome B16. En effet, après l'injection i.v. de cellules de B16 l'animal développe des "colonies" pulmonaires mélaniques assimilées à des métastases.
- nudes nu/nu SSCUP porteuses de mélanomes humains Beu et Dau.
- nudes nu/nu SSCUP porteuses de mélanome humain mélanique FOSS(ORIS).

Dans tous ces modèles humains et murins nous avons observé une bonne définition tumorale. Les images précoces sont plus difficiles à interpréter en raison d'une radioactivité pulmonaire et intraabdominale (digestive, rénale et urinaire) importante. Par contre à partir du temps 4 h les images tumorales sont de bonne qualité. Il est à noter que les zones de nécrose intratumorale ne sont pas visualisées. Nous avons pu obtenir des images de mélanome humain inférieur à 5 mm.

En ce qui concerne l'étude de spécificité nous n'avons obtenu aucune image après l'injection de A à des souris :

- BALB porteuses de la tumeur EMT6 (carcinome mammaire spontané de la souris)
- nudes nu/nu SSCUP porteuses d'hétérotransplants : SW 948 (adénocarcinome du côlon) et A 431 (tumeur de la vulve)

Une étude réalisée avec $^{123}I$-A a permis de confirmer les données obtenues en imagerie après l 'injection de $^{125}I$-A. Nous avons en effet pu observer des images identiques chez des souris porteuses du mélanome B16 et d'un hétérotransplant mélanique Beu et l'absence d'image chez les animaux porteurs de l'EMT6.

Des examens scintigraphiques ont aussi été réalisés après l'injection de B à des souris :

- C57 BL6 porteuses du mélanome B16
- nudes nu/nu SSCUP porteuses de l'hétérotransplant Beu

Nous avons pu observer une très bonne définition tumorale de 3 à 17 H après l'injection chez les animaux porteurs de ces deux modèles tumoraux.

Cette étude biologique montre que les dérivés selon l'invention sont des marqueurs intéressants du mélanome :

- leur toxicité est relativement faible,
- les autoradiographies donnent des images contrastées durables,
- la comparaison de la biodistribution chez les souris C57 BL6 porteuses du mélanome B16 est en faveur de leur utilisation pour l'imagerie. En effet, au niveau de la tumeur,les pourcentages de fixation et les rapports tissu/sang sont élevés jusqu'à la 8ème heure après l'injection.

Les composés marqués de l'invention sont donc particulièrement utiles comme agents radiophamaceutiques pour le diagnostic des mélanomes malins, mais pourront aussi être utilisés comme agents pour le traitement de ces mélanomes après marquage par $^{131}I$.

Par ailleurs, les dérivés de formule I porteurs d'isotopes stables tels que $^{19}F$ ou $^{13}C$ peuvent être utilisés pour l'imagerie en résonance magnétique nucléaire (I.R.M.).

C 57 BL 6 - B16

| Tissu \ Temps (h) | 1 | 2 | 4 | 6 | 8 | 12 | 24 |
|---|---|---|---|---|---|---|---|
| Sang | 1,04 ± 0,094 | 0,74 ± 0,045 | 0,54 ± 0,070 | 0,21 ± 0,020 | 0,34 ± 0,024 | 0,09 ± 0,032 | 0,022 ± 0,0012 |
| Plasma | 0,82 ± 0,068 | 0,55 ± 0,027 | 0,45 ± 0,031 | 0,21 ± 0,022 | 0,38 ± 0,023 | 0,11 ± 0,043 | 0,024 ± 0,0015 |
| Foie | 6,04 ± 0,396 | 4,02 ± 0,217 | 2,46 ± 0,519 | 0,95 ± 0,081 | 0,80 ± 0,106 | 0,25 ± 0,028 | 0,16 ± 0,005 |
| Rate | 5,91 ± 0,758 | 2,94 ± 0,517 | 1,36 ± 0,238 | 0,56 ± 0,162 | 0,46 ± 0,090 | 0,16 ± 0,075 | 0,02 ± 0,002 |
| Pancréas | 5,34 ± 0,412 | 3,42 ± 0,264 | 2,01 ± 0,432 | 0,55 ± 0,051 | 0,41 ± 0,091 | 0,13 ± 0,025 | 0,04 ± 0,0008 |
| Rein | 8,56 ± 0,430 | 5,93 ± 0,583 | 3,67 ± 0,578 | 1,10 ± 0,087 | 0,84 ± 0,155 | 0,19 ± 0,038 | 0,07 ± 0,002 |
| Intestin | 5,22 ± 0,232 | 3,94 ± 0,318 | 2,80 ± 0,586 | 0,75 ± 0,128 | 0,71 ± 0,115 | 0,14 ± 0,032 | 0,02 ± 0,002 |
| Estomac | 6,03 ± 0,751 | 3,87 ± 0,853 | 2,35 ± 0,593 | 1,14 ± 0,252 | 1,65 ± 0,532 | 0,19 ± 0,048 | 0,07 ± 0,002 |
| Poumons | 10,99 ± 0,967 | 6,12 ± 0,424 | 4,29 ± 0,853 | 1,99 ± 0,410 | 1,11 ± 0,197 | 0,20 ± 0,084 | 0,05 ± 0,004 |
| Oeil | 13,63 ± 1,053 | 12,98 ± 0,898 | 12,36 ± 0,903 | 12,13 ± 1,201 | 8,07 ± 0,294 | 8,49 ± 0,523 | 6,65 ± 0,282 |
| Cerveau | 2,67 ± 0,308 | 1,40 ± 0,074 | 0,70 ± 0,083 | 0,32 ± 0,032 | 0,18 ± 0,033 | 0,04 ± 0,011 | 0,005 ± 0,0006 |
| Glandes sous maxillaires | 6,06 ± 0,772 | 3,60 ± 0,299 | 3,68 ± 1,320 | 1,12 ± 0,108 | 0,98 ± 0,152 | 0,44 ± 0,207 | 0,09 ± 0,012 |
| Muscle | 1,55 ± 0,224 | 1,13 ± 0,064 | 0,66 ± 0,141 | 0,19 ± 0,021 | 0,14 ± 0,030 | 0,03 ± 0,007 | 0,009 ± 0,0004 |
| Tumeur | 6,75 ± 0,671 | 6,04 ± 0,471 | 4,82 ± 1,191 | 3,53 ± 0,307 | 3,42 ± 0,367 | 1,01 ± 0,393 | 0,79 ± 0,091 |

% DI/g

**A**

Tableau 1

**C 57 BL 6 - B16**

| Tissu \ Temps (h) | 1 | 2 | 4 | 6 | 8 | 12 | 24 |
|---|---|---|---|---|---|---|---|
| Plasma | 0,77 ± 0,034 | 0,74 ± 0,016 | 0,83 ± 0,081 | 1,00 ± 0,034 | 1,13 ± 0,025 | 1,17 ± 0,059 | 1,12 ± 0,033 |
| Foie | 5,88 ± 0,402 | 5,44 ± 0,157 | 4,37 ± 0,397 | 4,62 ± 0,198 | 2,29 ± 0,212 | 3,67 ± 0,580 | 7,47 ± 0,510 |
| Rate | 5,78 ± 0,804 | 3,91 ± 0,551 | 2,51 ± 0,437 | 2,83 ± 0,938 | 1,34 ± 0,266 | 2,36 ± 1,260 | 2,39 ± 1,127 |
| Pancréas | 5,30 ± 0,374 | 4,61 ± 0,166 | 3,55 ± 0,326 | 2,67 ± 0,180 | 1,21 ± 0,253 | 1,74 ± 0,183 | 1,88 ± 0,104 |
| Rein | 8,78 ± 0,111 | 7,93 ± 0,317 | 6,69 ± 0,725 | 5,42 ± 0,542 | 2,43 ± 0,397 | 2,56 ± 0,364 | 3,12 ± 0,168 |
| Intestin | 5,13 ± 0,400 | 5,31 ± 0,281 | 4,96 ± 0,398 | 3,63 ± 0,431 | 2,15 ± 0,249 | 1,75 ± 0,347 | 1,11 ± 0,022 |
| Estomac | 5,82 ± 0,721 | 5,18 ± 1,168 | 4,12 ± 0,525 | 5,38 ± 0,931 | 4,81 ± 1,227 | 2,42 ± 0,360 | 3,07 ± 0,137 |
| Poumons | 10,69 ± 0,954 | 8,30 ± 0,479 | 7,55 ± 0,565 | 8,78 ± 1,068 | 3,20 ± 0,485 | 2,19 ± 0,117 | 2,37 ± 0,133 |
| Oeil | 13,22 ± 0,776 | 17,51 ± 0,582 | 23,40 ± 1,943 | 59,48 ± 5,914 | 23,90 ± 1,899 | 129,62 ± 25,312 | 309,54 ± 22,214 |
| Cerveau | 2,54 ± 0,092 | 1,89 ± 0,020 | 1,28 ± 0,064 | 1,53 ± 0,063 | 0,52 ± 0,077 | 0,46 ± 0,045 | 0,25 ± 0,031 |
| Glandes sous-maxillaires | 5,92 ± 0,815 | 4,84 ± 0,237 | 6,16 ± 1,360 | 5,45 ± 0,282 | 2,83 ± 0,343 | 4,63 ± 0,745 | 4,26 ± 0,560 |
| Muscle | 1,46 ± 0,126 | 1,52 ± 0,030 | 1,17 ± 0,096 | 0,94 ± 0,078 | 0,40 ± 0,075 | 0,37 ± 0,033 | 0,38 ± 0,018 |
| Tumeur | 6,56 ± 0,595 | 8,23 ± 0,684 | 8,57 ± 1,215 | 17,66 ± 2,170 | 10,34 ± 1,775 | 17,12 ± 9,505 | 37,4 ± 7,04 |

$\frac{Tissu}{sang}$

**A**

Tableau 2

*Swiss nu/nu SSCUP - Beu*

| Tissu \ Temps (h) | 1 | 2 | 4 | 6 | 8 | 12 | 24 |
|---|---|---|---|---|---|---|---|
| **Sang** | 0,87 ± 0,024 | 0,53 ± 0,056 | 0,32 ± 0,032 | 0,13 ± 0,021 | 0,05 ± 0,011 | 0,02 ± 0,003 | 0,01 ± 0,002 |
| **Plasma** | 0,46 ± 0,022 | 0,35 ± 0,057 | 0,20 ± 0,021 | 0,12 ± 0,034 | 0,04 ± 0,013 | 0,02 ± 0,002 | 0,01 ± 0,002 |
| **Foie** | 5,27 ± 0,220 | 3,50 ± 0,332 | 2,28 ± 0,164 | 0,94 ± 0,086 | 0,52 ± 0,042 | 0,28 ± 0,033 | 0,13 ± 0,019 |
| **Rate** | 4,11 ± 0,217 | 2,12 ± 0,292 | 1,07 ± 0,023 | 0,28 ± 0,029 | 0,11 ± 0,015 | 0,04 ± 0,006 | 0,02 ± 0,002 |
| **Pancréas** | 3,99 ± 0,104 | 2,28 ± 0,270 | 1,39 ± 0,123 | 0,36 ± 0,039 | 0,12 ± 0,010 | 0,06 ± 0,011 | 0,05 ± 0,004 |
| **Rein** | 7,63 ± 0,227 | 5,90 ± 0,691 | 4,28 ± 0,611 | 0,99 ± 0,140 | 0,28 ± 0,0029 | 0,10 ± 0,024 | 0,06 ± 0,016 |
| **Intestin** | 3,76 ± 0,348 | 2,35 ± 0,299 | 1,56 ± 0,204 | 0,52 ± 0,119 | 0,15 ± 0,020 | 0,08 ± 0,033 | 0,03 ± 0,009 |
| **Estomac** | 2,49 ± 0,074 | 1,51 ± 0,239 | 1,16 ± 0,123 | 0,49 ± 0,067 | 0,22 ± 0,025 | 0,07 ± 0,022 | 0,04 ± 0,006 |
| **Poumons** | 7,77 ± 0,172 | 4,71 ± 0,622 | 3,60 ± 0,272 | 1,25 ± 0,154 | 0,35 ± 0,058 | 0,08 ± 0,023 | 0,05 ± 0,009 |
| **Oeil** | 0,64 ± 0,041 | 0,56 ± 0,135 | 0,34 ± 0,007 | 0,13 ± 0,009 | 0,07 ± 0,005 | 0,03 ± 0,002 | 0,03 ± 0,005 |
| **Cerveau** | 1,66 ± 0,103 | 1,13 ± 0,134 | 0,78 ± 0,054 | 0,28 ± 0,026 | 0,13 ± 0,019 | 0,01 ± 0,002 | 0,006 ± 0,0012 |
| **Glandes sous-maxillaires** | 5,11 ± 0,346 | 2,90 ± 0,275 | 1,71 ± 0,177 | 0,95 ± 0,255 | 0,24 ± 0,042 | 0,04 ± 0,007 | 0,03 ± 0,004 |
| **Muscle** | 1,30 ± 0,050 | 0,93 ± 0,144 | 0,51 ± 0,046 | 0,33 ± 0,063 | 0,08 ± 0,015 | 0,01 ± 0,002 | 0,01 ± 0,002 |
| **Tumeur** | 3,51 ± 0,397 | 3,17 ± 0,405 | 3,25 ± 0,172 | 1,96 ± 0,241 | 1,07 ± 0,159 | 0,36 ± 0,050 | 0,07 ± 0,005 |

*% DI/g*

A Tableau 3

*Swiss nu/nu SSCUP - Beu*

| Tissu \ Temps (h) | 1 | 2 | 4 | 6 | 8 | 12 | 24 |
|---|---|---|---|---|---|---|---|
| **Plasma** | 0,53 ± 0,022 | 0,66 ± 0,064 | 0,63 ± 0,010 | 0,95 ± 0,059 | 0,99 ± 0,047 | 1,06 ± 0,106 | 1,06 ± 0,169 |
| **Foie** | 6,07 ± 0,267 | 6,66 ± 0,297 | 7,37 ± 0,374 | 7,68 ± 1,136 | 11,47 ± 1,690 | 16,09 ± 1,576 | 10,48 ± 0,947 |
| **Rate** | 4,72 ± 0,216 | 4,00 ± 0,352 | 3,58 ± 0,393 | 2,31 ± 0,394 | 2,45 ± 0,495 | 2,11 ± 0,121 | 2,01 ± 0,219 |
| **Pancréas** | 4,59 ± 0,098 | 4,28 ± 0,104 | 4,47 ± 0,222 | 2,97 ± 0,397 | 3,00 ± 2,585 | 3,10 ± 0,290 | 3,70 ± 0,447 |
| **Rein** | 8,95 ± 0,306 | 11,17 ± 0,736 | 13,17 ± 0,980 | 6,62 ± 1,281 | 6,29 ± 1,071 | 5,52 ± 0,592 | 4,79 ± 0,428 |
| **Intestin** | 4,31 ± 0,363 | 4,52 ± 0,536 | 5,05 ± 0,576 | 3,57 ± 0,814 | 3,51 ± 0,709 | 4,00 ± 1,449 | 2,31 ± 0,291 |
| **Estomac** | 2,88 ± 0,170 | 2,84 ± 0,260 | 3,71 ± 0,252 | 3,75 ± 0,262 | 4,74 ± 0,687 | 3,44 ± 0,495 | 3,06 ± 0,265 |
| **Poumons** | 8,95 ± 0,152 | 8,75 ± 0,319 | 11,74 ± 1,029 | 8,76 ± 1,714 | 7,60 ± 1,088 | 4,06 ± 0,117 | 3,45 ± 0,151 |
| **Oeil** | 0,74 ± 0,042 | 1,04 ± 0,178 | 1,13 ± 0,131 | 1,07 ± 0,117 | 1,69 ± 0,287 | 1,90 ± 0,300 | 2,38 ± 0,712 |
| **Cerveau** | 1,89 ± 0,094 | 2,13 ± 0,070 | 2,51 ± 0,173 | 1,87 ± 0,610 | 2,94 ± 0,644 | 0,94 ± 0,085 | 0,44 ± 0,034 |
| **Glandes sous-maxillaires** | 5,86 ± 0,297 | 5,53 ± 0,296 | 5,46 ± 0,282 | 6,73 ± 0,863 | 5,04 ± 0,504 | 2,33 ± 0,179 | 2,80 ± 0,207 |
| **Muscle** | 1,50 ± 0,052 | 1,75 ± 0,141 | 1,65 ± 0,083 | 2,08 ± 0,315 | 1,78 ± 0,514 | 0,80 ± 0,089 | 0,91 ± 0,131 |
| **Tumeur** | 4,08 ± 0,508 | 6,26 ± 0,947 | 9,70 ± 1,018 | 16,30 ± 4,185 | 24,43 ± 5,417 | 19,93 ± 2,180 | 6,41 ± 2,022 |

Tissu/sang

A

Tableau 4

Swiss nu/nu SSCUP - Dau

| Tissu \ Temps (h.) | 1 | 2 | 4 | 6 | 8 | 12 | 24 |
|---|---|---|---|---|---|---|---|
| Sang | 0,89 ± 0,054 | 0,52 ± 0,044 | 0,29 ± 0,025 | 0,23 ± 0,019 | 0,14 ± 0,042 | 0,06 ± 0,007 | 0,008 ± 0,0003 |
| Plasma | 0,55 ± 0,039 | 0,33 ± 0,035 | 0,24 ± 0,030 | 0,24 ± 0,025 | 0,15 ± 0,055 | 0,06 ± 0,006 | 0,007 ± 0,0005 |
| Foie | 5,68 ± 0,621 | 3,35 ± 0,262 | 2,10 ± 0,281 | 1,10 ± 0,045 | 0,71 ± 0,085 | 0,3 ± 0,015 | 0,1 ± 0,006 |
| Rate | 3,48 ± 0,178 | 1,95 ± 0,097 | 0,80 ± 0,078 | 0,30 ± 0,023 | 0,16 ± 0,017 | 0,07 ± 0,006 | 0,02 ± 0,008 |
| Pancréas | 4,06 ± 0,208 | 2,29 ± 0,101 | 0,97 ± 0,108 | 0,41 ± 0,028 | 0,24 ± 0,024 | 0,13 ± 0,010 | 0,03 ± 0,004 |
| Rein | 8,39 ± 0,908 | 5,12 ± 0,456 | 2,15 ± 0,296 | 0,78 ± 0,034 | 0,32 ± 0,059 | 0,14 ± 0,011 | 0,03 ± 0,004 |
| Intestin | 3,74 ± 0,340 | 2,67 ± 0,328 | 1,10 ± 0,174 | 0,42 ± 0,042 | 0,38 ± 0,101 | 0,27 ± 0,118 | 0,01 ± 0,002 |
| Estomac | 2,32 ± 0,172 | 1,85 ± 0,173 | 1,36 ± 0,349 | 1,17 ± 0,025 | 0,71 ± 0,182 | 0,38 ± 0,093 | 0,03 ± 0,004 |
| Poumons | 6,88 ± 0,840 | 5,63 ± 0,655 | 2,84 ± 0,393 | 0,76 ± 0,092 | 0,45 ± 0,082 | 0,11 ± 0,009 | 0,02 ± 0,004 |
| Oeil | 0,68 ± 0,146 | 0,54 ± 0,097 | 0,31 ± 0,039 | 0,16 ± 0,010 | 0,10 ± 0,012 | 0,04 ± 0,004 | 0,02 ± 0,009 |
| Cerveau | 1,40 ± 0,114 | 0,99 ± 0,081 | 0,51 ± 0,060 | 0,20 ± 0,011 | 0,12 ± 0,008 | 0,02 ± 0,002 | 0,002 ± 0,0003 |
| Glandes sous-maxillaires | 4,75 ± 0,467 | 3,56 ± 0,280 | 1,50 ± 0,144 | 0,86 ± 0,124 | 0,77 ± 0,291 | 0,30 ± 0,040 | 0,02 ± 0,002 |
| Muscle | 1,28 ± 0,092 | 0,79 ± 0,064 | 0,32 ± 0,035 | 0,11 ± 0,003 | 0,07 ± 0,011 | 0,02 | 0,006 ± 0,0005 |
| Tumeur | 1,51 ± 0,074 | 1,29 ± 0,299 | 0,45 ± 0,033 | 0,23 ± 0,024 | 0,11 ± 0,031 | 0,07 ± 0,024 | 0,009 ± 0,0005 |

% DI/g

A

Tableau 5

*Swiss nu/nu SSCUP - Dau*

| Tissu \ Temps (h) | 1 | 2 | 4 | 6 | 8 | 12 | 24 |
|---|---|---|---|---|---|---|---|
| Plasma | 0,62 ± 0,044 | 0,64 ± 0,038 | 0,82 ± 0,052 | 1,06 ± 0,019 | 1,07 ± 0,066 | 1,01 ± 0,077 | 0,97 ± 0,042 |
| Foie | 6,37 ± 0,593 | 6,47 ± 0,246 | 7,16 ± 0,528 | 4,91 ± 0,502 | 6,06 ± 1,210 | 5,19 ± 0,621 | 13,18 ± 1,199 |
| Rate | 3,91 ± 0,051 | 3,83 ± 0,280 | 2,76 ± 0,205 | 1,37 ± 0,157 | 1,43 ± 0,375 | 1,22 ± 0,135 | 2,59 ± 1,002 |
| Pancréas | 4,56 ± 0,058 | 4,47 ± 0,250 | 3,64 ± 0,523 | 1,85 ± 0,189 | 2,06 ± 0,526 | 2,29 ± 0,215 | 3,82 ± 0,484 |
| Rein | 9,13 ± 0,717 | 9,83 ± 0,360 | 7,46 ± 0,952 | 3,37 ± 0,421 | 2,9 ± 0,956 | 2,58 ± 0,433 | 4,6 ± 0,667 |
| Intestin | 4,23 ± 0,427 | 5,04 ± 0,290 | 3,75 ± 0,462 | 1,77 ± 0,237 | 2,93 ± 0,667 | 4,47 ± 1,625 | 1,52 ± 0,301 |
| Estomac | 2,63 ± 0,241 | 3,58 ± 0,209 | 4,50 ± 0,788 | 5,01 ± 0,598 | 5,16 ± 0,918 | 6,25 ± 0,1264 | 3,39 ± 0,540 |
| Poumons | 7,71 ± 0,767 | 10,92 ± 0,815 | 9,94 ± 1,404 | 3,34 ± 0,388 | 3,96 ± 0,961 | 1,84 ± 0,135 | 3,45 ± 0,597 |
| Oeil | 0,74 ± 0,113 | 1,02 ± 0,127 | 1,1 ± 0,093 | 0,73 ± 0,033 | 0,78 ± 0,130 | 0,72 ± 0,033 | 2,99 ± 1,419 |
| Cerveau | 1,56 ± 0,065 | 1,91 ± 0,110 | 1,80 ± 0,209 | 0,91 ± 0,082 | 1,06 ± 0,278 | 0,44 ± 0,044 | 0,28 ± 0,039 |
| Glandes sous-maxillaires | 5,32 ± 0,379 | 6,97 ± 0,600 | 5,19 ± 0,382 | 3,70 ± 0,373 | 5,14 ± 0,541 | 21 ± 0,945 | 3,13 ± 0,281 |
| Muscle | 1,44 ± 0,087 | 1,53 ± 0,082 | 1,12 ± 0,110 | 0,49 ± 0,038 | 0,67 ± 0,181 | 0,36 ± 0,039 | 0,82 ± 0,064 |
| Tumeur | 1,70 ± 0,036 | 2,47 ± 0,487 | 1,59 ± 0,140 | 0,98 ± 0,080 | 1,43 ± 0,329 | 1,36 ± 0,528 | 1,17 ± 0,108 |

Tissu / sang

A

Tableau 6

Tableau 7

C57 BL6 - B16

| % D I /g tissu \ Temps (h) | 1 | 2 | 4 | 6 | 24 |
|---|---|---|---|---|---|
| Sang | 1,31 | 0,67 | 0,27 | 0,14 | 0,03 |
| Plasma | 0,73 | 0,40 | 0,21 | 0,13 | 0,02 |
| Foie | 6,96 | 3,72 | 2,05 | 0,96 | 0,22 |
| Rein | 14,37 | 3,64 | 4,42 | 1,86 | 0,16 |
| Poumons | 14,42 | 8,00 | 4,49 | 2,10 | 0,05 |
| Oeil* | 0,45 | 0,43 | 0,42 | 0,38 | 0,24 |
| Cerveau | 3,26 | 2,09 | 1,17 | 0,50 | 0,01 |
| Muscle | 2,55 | 1,33 | 0,67 | 0,20 | 0,02 |
| Tumeur | 6,03 | 5,73 | 4,36 | 4,69 | 0,49 |

B

*Tableau 8*

**C57 BL6 - B16**

| Tissu/sang \ Temps (h) | 1 | 2 | 4 | 6 | 24 |
|---|---|---|---|---|---|
| **Plasma** | 0,56 | 0.60 | 0,79 | 0,90 | 0,77 |
| **Foie** | 5,32 | 5.55 | 7,58 | 6,80 | 7,49 |
| **Rein** | 10,92 | 12.82 | 16,38 | 13,04 | 5,24 |
| **Poumons** | 11,00 | 11.94 | 16,64 | 14.66 | 1,74 |
| **Oeil** | 9,70 | 17.50 | 39.70 | 73 | 200 |
| **Cerveau** | 2.48 | 3.11 | 4,33 | 3.51 | 0.46 |
| **Muscle** | 1,95 | 1.96 | 2.49 | 1.47 | 0.75 |
| **Tumeur** | 4,70 | 8.51 | 16.17 | 36 | 16 |

**B**

Tableau 9

Swiss nu/nu SSCUP - Dau

| Tissu \ Temps (h.) | 1 | 2 | 4 | 6 | 8 | 12 | 24 |
|---|---|---|---|---|---|---|---|
| Sang | 0,89 ± 0,033 | 0,93 ± 0,099 | 0,30 ± 0,053 | 0,53 ± 0,083 | 0,11 ± 0,016 | 0,015 ± 0,0024 | 0,009 ± 0,0005 |
| Plasma | 0,71 ± 0,038 | 0,85 ± 0,15 | 0,28 ± 0,049 | 0,61 ± 0,047 | 0,13 ± 0,017 | 0,02 ± 0,002 | 0,009 ± 0,0008 |
| Foie | 4,95 ± 0,177 | 3,16 ± 0,39 | 1,52 ± 0,201 | 0,74 ± 0,054 | 0,63 ± 0,082 | 0,20 ± 0,013 | 0,19 ± 0,008 |
| Rate | 2,54 ± 0,114 | 2,02 ± 0,17 | 0,62 ± 0,135 | 0,33 ± 0,030 | 0,14 ± 0,022 | 0,05 ± 0,005 | 0,09 ± 0,009 |
| Pancréas | 2,86 ± 0,127 | 2,13 ± 0,17 | 0,83 ± 0,185 | 0,40 ± 0,026 | 0,14 ± 0,034 | 0,08 ± 0,005 | 0,01 |
| Rein | 8,15 ± 0,499 | 6,17 ± 0,46 | 2,51 ± 0,589 | 1,07 ± 0,164 | 0,42 ± 0,144 | 0,08 ± 0,005 | 0,04 ± 0,002 |
| Intestin | 3,10 ± 0,314 | 2,57 ± 0,16 | 1,19 ± 0,218 | 1,05 ± 0,120 | 0,36 ± 0,057 | 0,05 ± 0,006 | 0,01 ± 0,0004 |
| Estomac | 2,38 ± 0,154 | 3,03 ± 0,547 | 1,23 ± 0,201 | 2 ± 0,253 | 0,58 ± 0,098 | 0,10 ± 0,009 | 0,02 ± 0,002 |
| Poumons | 5,35 ± 0,319 | 5,56 ± 0,598 | 2,63 ± 0,485 | 0,93 ± 0,038 | 0,43 ± 0,143 | 0,04 ± 0,004 | 0,02 ± 0,002 |
| Oeil | 0,51 ± 0,028 | 0,50 ± 0,024 | 0,21 ± 0,021 | 0,19 ± 0,012 | 0,06 ± 0,012 | 0,01 ± 0,002 | 0,008 ± 0,0010 |
| Cerveau | 1,28 ± 0,079 | 1,25 ± 0,142 | 0,63 ± 0,101 | 0,22 ± 0,020 | 0,14 ± 0,035 | 0,01 ± 0,002 | 0,002 ± 0,0002 |
| Glandes sous-maxillaires | 4,28 ± 0,207 | 4,14 ± 0,370 | 1,68 ± 0,351 | 2,74 ± 0,492 | 0,39 ± 0,046 | 0,04 ± 0,005 | 0,02 ± 0,002 |
| Muscle | 1,04 ± 0,040 | 0,86 ± 0,101 | 0,41 ± 0,120 | 0,12 ± 0,026 | 0,07 ± 0,014 | 0,008 ± 0,0013 | 0,002 ± 0,0002 |
| Tumeur | 1,19 ± 0,048 | 1,27 ± 0,086 | 0,93 ± 0,425 | 0,34 ± 0,020 | 0,12 ± 0,024 | 0,02 | 0,007 ± 0,0006 |

% DI/g

B

*Swiss nu/nu SSCUP - Dau*

Tableau 10

| Temps (h.) / Tissu | 1 | 2 | 4 | 6 | 8 | 12 | 24 |
|---|---|---|---|---|---|---|---|
| **Plasma** | 0,80 ± 0,017 | 0,89 ± 0,068 | 0,94 ± 0,058 | 1,16 ± 0,016 | 1,19 ± 0,020 | 1,16 ± 0,037 | 1,07 ± 0,154 |
| **Foie** | 5,56 ± 0,225 | 3,62 ± 0,68 | 5,21 ± 0,519 | 1,43 ± 0,139 | 6,03 ± 0,678 | 13,34 ± 0,462 | 21,88 ± 1,493 |
| **Rate** | 2,84 ± 0,091 | 2,31 ± 0,36 | 2,07 ± 0,256 | 0,62 ± 0,045 | 1,28 ± 0,158 | 3,68 ± 0,493 | 11,17 ± 1,75 |
| **Pancréas** | 3,20 ± 0,099 | 2,42 ± 0,36 | 2,70 ± 0,300 | 0,76 ± 0,050 | 1,22 ± 0,151 | 5 ± 0,310 | 1,40 ± 0,120 |
| **Rein** | 9,10 ± 0,286 | 7,00 ± 1,03 | 8,03 ± 0,813 | 2,01 ± 0,252 | 3,72 ± 0,573 | 5,46 ± 0,217 | 4,64 ± 0,328 |
| **Intestin** | 3,44 ± 0,231 | 2,87 ± 0,34 | 4,08 ± 0,599 | 1,97 ± 0,112 | 3,30 ± 0,269 | 3,20 ± 0,502 | 1,16 ± 0,141 |
| **Estomac** | 2,66 ± 0,101 | 3,40 ± 0,694 | 4,34 ± 0,924 | 3,78 ± 0,400 | 5,35 ± 0,355 | 7,03 ± 0,728 | 2,28 ± 0,165 |
| **Poumons** | 6 ± 0,353 | 6,33 ± 1,166 | 8,77 ± 0,838 | 1,82 ± 0,187 | 3,69 ± 0,585 | 3,04 ± 0,401 | 1,72 ± 0,182 |
| **Oeil** | 0,57 ± 0,011 | 0,55 ± 0,052 | 0,71 ± 0,056 | 0,36 ± 0,023 | 0,60 ± 0,040 | 0,88 ± 0,081 | 0,94 ± 0,213 |
| **Cerveau** | 1,44 ± 0,069 | 1,43 ± 0,261 | 2,21 ± 0,304 | 0,44 ± 0,060 | 1,31 ± 0,180 | 0,74 ± 0,072 | 0,22 ± 0,029 |
| **Glandes sous-maxillaires** | 4,82 ± 0,285 | 4,48 ± 0,220 | 5,44 ± 0,297 | 5,16 ± 0,853 | 3,65 ± 0,266 | 2,52 ± 0,236 | 1,81 ± 0,169 |
| **Muscle** | 1,17 ± 0,040 | 0,98 ± 0,182 | 1,24 ± 0,153 | 0,24 ± 0,056 | 0,74 ± 0,216 | 0,45 ± 0,111 | 0,27 ± 0,043 |
| **Tumeur** | 1,33 ± 0,015 | 1,39 ± 0,103 | 2,68 ± 0,757 | 0,65 ± 0,025 | 1,17 ± 0,314 | 1,11 ± 0,059 | 0,79 ± 0,092 |

**B**

$\frac{Tissu}{sang}$

## Revendications

**1.** Utilisation d'un composé répondant à la formule générale

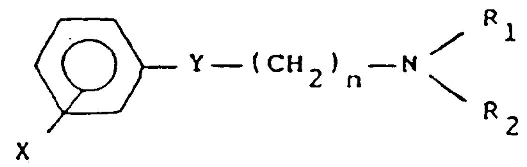

(I)

dans laquelle
X représente un atome d'hydrogène ou d'halogène,
Y représente un groupement Z-CONH-, Z-O-, Z-S(O)-$_{n1}$ avec $n_1$ = 0, 1, 2 ou 3, Z-$SO_2$-NH- ou Z-NHCO-, Z étant une liaison, -$CH_2$ - ou

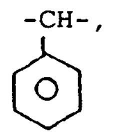

n est un entier de 2 à 4, et
$R_1$ et $R_2$ représentent chacun un atome d'hydrogène, un groupe alkyle en $C_1$ à $C_6$, aryle ou aralkyle en $C_6$ à $C_{12}$, ces composés pouvant être marqués par des radioisotopes choisis parmi $^{123}I$, $^{125}I$, $^{131}I$, $^{75}Br$, $^{77}Br$, $^{18}F$, ou $^{11}C$, ou des isotopes stables choisis parmi $^{13}C$ et $^{19}F$,
ainsi que leurs sels d'addition à des acides pharmaceutiquement acceptables, pour la préparation d'un agent pour le diagnostic et/ou le traitement des mélanomes malins.

**2.** Utilisation selon la revendication 1, d'un composé répondant à la formule (I) dans laquelle X représente $^{123}I$, $^{125}I$, $^{131}I$, Y représente Z-CONH- ou Z-$SO_2$-NH-, Z étant une liaison, -$CH_2$- ou

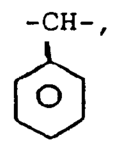

et
$R_1$ et $R_2$ représentent chacun un atome d'hydrogène, ou un groupe alkyle en $C_1$ à $C_6$.

**3.** Utilisation du composé suivant pour la préparation d'un agent radiopharmaceutique pour le diagnostic et le traitement des mélanomes malins, répondant à la formule

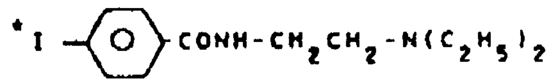

*I étant $^{125}I$, $^{123}I$, $^{131}I$.

**4.** Utilisation d'un composé répondant à la formule générale

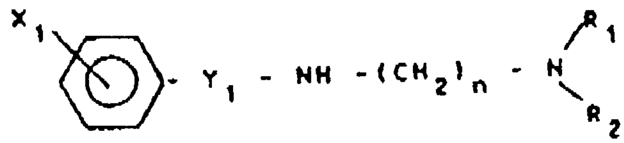

(II)

dans laquelle
$X_1$ représente un atome d'halogène ou un de ses radioiso- topes,
$Y_1$ représente un groupe CO ou $SO_2$,
n est un entier de 2 à 4, et
$R_1$ et $R_2$ représentent chacun un atome d'hydrogène, ou un groupe alkyle en $C_1$ à $C_6$,
ainsi que leurs sels d'addition à des acides pharmaceuti- quement acceptables, pour la préparation d'un agent pour le diagnostic et/ou le traitement des mélanomes malins.

**5.** Agents pharmaceutiques répondant à la formule générale

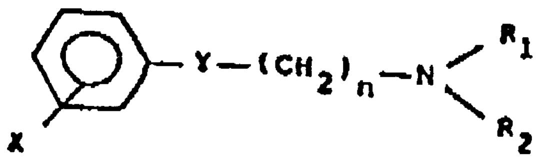

(I)

selon la revendication 1,
dans laquelle
X représente un atome d'hydrogène ou d'halogène,
Y représente un groupement Z-CONH-, Z-O-, Z-S(O)-$_{n1}$ avec $n_1$ =0, 1, 2 ou 3, Z-$SO_2$-NH- ou Z-NHCO-,
Z étant une liaison, -$CH_2$- ou

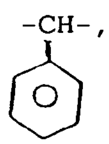

n est un entier de 2 à 4, et
$R_1$ et $R_2$ représentent chacun un atome d'hydrogène, un groupe alkyle en $C_1$ à $C_6$, aryle ou aralkyle en $C_6$ à $C_{12}$, ces composés pouvant être marqués par des radioisotopes choisis parmi $^{123}I$, $^{125}I$, $^{131}I$, $^{75}Br$, $^{77}Br$, $^{18}F$, ou $^{11}C$, ou des isotopes stables choisis parmi $^{13}C$ et $^{19}F$,
ainsi que leurs sels d'addition à des acides pharmaceuti- quement acceptables, à l'exclusion des composés de formule (I), dans laquelle Y représente Z-CONH-, Z étant une liaison, n représente 2, et $R_1$ et $R_2$ représentent simultanément un atome d'hydrogène, des composés de formule (I), dans laquelle Y représente Z-CONH-, Z étant une liaison, et X représente F, Cl, Br en position ortho, méta ou para ou I en position ortho ou méta, des composés de formule (I) dans laquelle Y représente Z-$SO_2$-NH, Z étant une liaison, et X représente Cl en position para et du composé de formule (I) dans laquelle Y repré- sente Z-CO-NH-, Z étant une liaison, X est un atome d'iode en position para, et $R_1$ et $R_2$ représentent un groupe éthyle.

**6.** Agents selon la revendication 5, répondant à la formule (I) dans laquelle X représente $^{123}I$, $^{125}I$ ou $^{131}I$, Y représente Z-CONH ou Z-$SO_2$-NH-, Z étant une liaison, -$CH_2$- ou

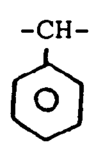

et

$R_1$ et $R_2$ représentent chacun un atome d'hydrogène, ou un groupe alkyle en $C_1$ à $C_6$.

7. Agents selon la revendication 5, répondant à la formule générale

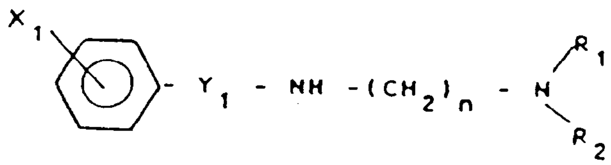

(II)

dans laquelle
$X_1$ représente un atome d'halogène ou un de ses radioiso- topes,
$Y_1$ représente un groupe CO ou $SO_2$,
n est un entier de 2 à 4, et
$R_1$ et $R_2$ représentent chacun un atome d'hydrogène, ou un groupe alkyle en $C_1$ à $C_6$,
ainsi que leurs sels d'addition à des acides pharmaceuti- quement acceptables, à l'exclusion des composés de formule (II) dans laquelle $Y_1$ représente CO, n représente 2, et $R_1$ et $R_2$ représentent simultanément l'hydrogène, des composés de formule (II), dans laquelle $Y_1$ représente CO, $X_1$ représente F, Cl, Br en position ortho, méta ou para ou 1 en position ortho ou méta, des composés de formule (II) dans laquelle $Y_1$ représente $SO_2$ et $X_1$ repré- sente Cl en position para et du composé de formule (II) dans laquelle $Y_1$ représente CO, $X_1$ est un atome d'iode en position para, et $R_1$ et $R_2$ représentent un groupe éthyle.

8. Agent pharmaceutique selon la revendica- tion 5, caractérisé en ce qu'il consiste en le N-(éthylamino-2-éthyl) iodo-4-benzamide et son chlorhy- drate.

9. Agent pharmaceutique selon la revendica- tion 5, caractérisé en ce qu'il consiste en le N-(diméthylamino-2-éthyl) iodo-4-benzamide .

10. Agent pharmaceutique selon la revendica- tion 5, caractérisé en ce qu'il consiste en le N-(méthylamino-2-éthyl) iodo-4-benzamide .

11. Agent pharmaceutique selon la revendica- tion 5, caractérisé en ce qu'il consiste en le N-(diisopropylamino-2-éthyl) iodo-4-benzamide .

12. Agent pharmaceutique selon la revendica- tion 5, caractérisé en ce qu'il consiste en le N-(isopropylamino-2-éthyl) iodo-4-benzamide .

13. Agent pharmaceutique selon la revendica- tion 5, caractérisé en ce qu'il consiste en le N-(diméthylamino-3-propyl) iodo-4-benzamide .

14. Agent pharmaceutique selon la revendica- tion 5, caractérisé en ce qu'il consiste en le N-(méthylamino-3-propyl) iodo-4-benzamide .

15. Agent pharmaceutique selon la revendica- tion 5, caractérisé en ce qu'il consiste en le N-(diéthylamino-2-éthyl) iodo-4-benzènesulfonamide.

16. Utilisation du N-(diéthylamino-2-éthyl) iodo-2-benzamide pour la préparation d'un agent radio- pharmaceutique pour le diagnostic et le traitement des mélanomes malins.

17. Utilisation du N-(diéthylamino-3-propyl) iodo-4-benzamide pour la préparation d'un agent radio- pharmaceutique pour le diagnostic et le traitement des mélanomes malins.

18. Utilisation du N-(éthylamino-2-éthyl) iodo-4-benzamide ou son chlorhydrate pour la préparation d'un agent radiopharmaceutique pour le diagnostic et le traitement des mélanomes malins .

19. Utilisation du N-(diméthylamino-2-éthyl) iodo-4-benzamide pour la préparation d'un agent radio- pharmaceutique pour le diagnostic et le traitement des mélanomes malins .

20. Utilisation du N-(méthylamino-2-éthyl) iodo-4-benzamide pour la préparation d'un agent radio- pharmaceutique pour le diagnostic et le traitement des mélanomes malins .

21. Utilisation du N-(diisopropylamino-2- éthyl) iodo-4-benzamide pour la préparation d'un agent radiopharmaceutique pour le diagnostic et le traitement des mélanomes malins .

22. Utilisation du N-(isopropylamino-2-éthyl) iodo-4-benzamide pour la préparation d'un agent radio- pharmaceutique pour le diagnostic et le traitement des mélanomes malins .

23. Utilisation du N-(diméthylamino-3-propyl) iodo-4-benzamide pour la préparation d'un agent radio- pharmaceutique pour le diagnostic et le traitement des mélanomes malins .

24. Utilisation du N-(méthylamino-3-propyl) iodo-4-benzamide pour la préparation d'un agent radio- pharmaceutique pour le diagnostic et le traitement des mélanomes malins .

25. Utilisation du N-(diéthylamino-2-éthyl) iodo-4-benzènesulfonamide pour la préparation d'un agent radiopharmaceutique pour le diagnostic et le traitement des mélanomes malins .

26. Utilisation de l'(amino-2-éthyl) iodo 4-benzamide pour la préparation d'un agent radiopharmaceu- tique pour le diagnostic et le traitement des mélanomes malins .

27. Agents marqués par des isotopes stables choisis parmi $^{13}C$ et $^{19}F$ selon la revendication 5 pour l'utilisation dans l'imagerie en résonnance magnétique nucléaire.

## Claims

1. Use of a compound corresponding to the general formula

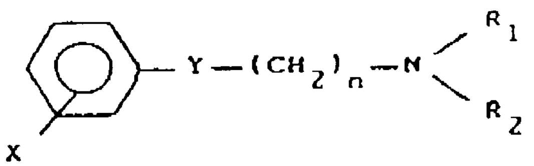

(I)

wherein
X represents a hydrogen or halogen atom,
Y represents a Z-CONH-, Z-O- or Z-S(O)$_{n1}$- group wherein $n_1$ = 0, 1, 2, or 3, or a Z-$SO_2$-NH- or Z-NHCO- group wherein Z represents a bond, -$CH_2$- or

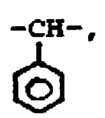

n is an integer from 2 to 4, and
$R_1$ and $R_2$ each represent a hydrogen atom, a $C_1$ to $C_6$ alkyl group or a $C_6$ to $C_{12}$ aryl or aralkyl group, these compounds optionally being labelled with radioisotopes selected from $^{123}I$, $^{125}I$, $^{131}I$, $^{75}Br$, $^{77}Br$, $^{18}F$, or $^{11}C$, or stable isotopes selected from $^{13}C$ and $^{19}F$
as well as the addition salts thereof with pharmaceutically acceptable acids, for the preparation of an agent for diagnosing and/or treating malignant melanomas.

2. Use according to Claim 1 of a compound corresponding to formula (I) wherein X represents $^{123}I$, $^{125}I$, $^{131}I$, Y represents Z-CONH-, or Z-$SO_2$-NH-, Z being a bond, -$CH_2$- or

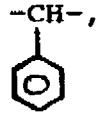

and
$R_1$ and $R_2$ each represent a hydrogen atom, or a $C_1$ to $C_6$ alkyl group.

3. Use of the following compound for preparing a radiopharmaceutical agent for diagnosing and treating malignant melanomas, corresponding to the formula

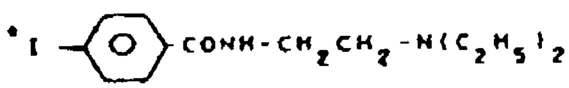

* I denotes $^{125}I$, $^{123}I$ or $^{131}I$

4. Use of a compound corresponding to the general formula

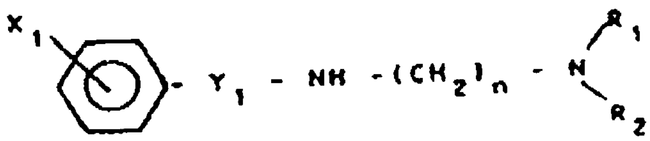

(II)

wherein
$X_1$ denotes a halogen atom or one of its radioisotopes,
$Y_1$ denotes a CO or $SO_2$ group,
n is an integer from 2 to 4, and
$R_1$ and $R_2$ each represent a hydrogen atom or a $C_1$ to $C_6$ alkyl group,
and the addition salts thereof with pharmaceutically acceptable acids, for preparing an agent for the diagnosis and/or treatment of malignant melanomas.

5. Pharmaceutical agents corresponding to the general formula

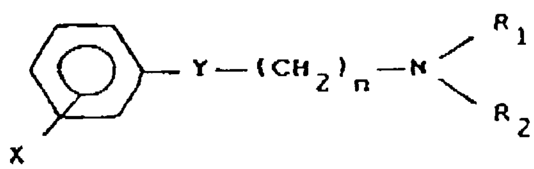

(I)

according to claim 1, wherein
X denotes a hydrogen or halogen atom,
Y denotes a Z-CONH-, Z-O- or Z-S(O)$_{n1}$- group wherein $n_1$ = 0, 1, 2 or 3, or a Z-$SO_2$-NH- or Z-NHCO- group, wherein Z denotes a bond, -$CH_2$- or

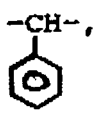

n is an integer from 2 to 4, and
$R_1$ and $R_2$ each denote a hydrogen atom, a $C_1$ to $C_6$ alkyl group or a $C_6$ to $C_{12}$ aryl or aralkyl group, these compounds optionally being labelled by radioisotopes selected from $^{123}I$, $^{125}I$, $^{131}I$, $^{75}Br$, $^{77}Br$, $^{18}F$ or $^{11}C$, or stable isotopes selected from $^{13}C$ and $^{19}F$,
and the addition salts thereof with pharmaceutical acceptable acids, with the exception of compounds of formula I wherein Y denotes Z-CONH-, Z being a bond, n represents 2, and $R_1$ and $R_2$ simultaneously denote a hydrogen atom, compounds of formula (I) wherein Y denotes Z-CONH-, Z being a bond, and X represents F, Cl, Br in the ortho, meta or para position or I in the ortho or meta position, compounds of formula (I) wherein Y denotes Z-$SO_2$-NH-, Z being a bond and X represents Cl in the para position and the compound of formula (I) wherein Y denotes Z-CONH-, Z being a bond, X is an iodine atom in the para position and $R_1$ and $R_2$ represent an ethyl group.

**6.** Agents according to Claim 5, corresponding to the formula (I) wherein X denotes $^{123}I$, $^{125}I$, $^{131}I$, Y represents Z-CONH- or Z-$SO_2$-NH-, Z being a bond, -$CH_2$- or

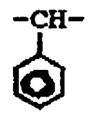

and
$R_1$ and $R_2$ each denote a hydrogen atom, or a $C_1$ to $C_6$ alkyl group.

**7.** Agents according to Claim 5, corresponding to the general formula

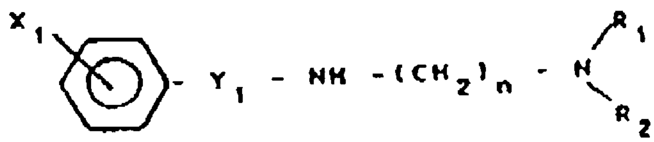 (II)

wherein $X_1$ denotes a halogen atom or one of its radioisotopes,
$Y_1$ represents a CO or $SO_2$ group,
n is an integer from 2 to 4, and
$R_1$ and each represent a hydrogen atom or a $C_1$ to $C_6$ alkyl group, as well as the addition salts thereof with pharmaceutically acceptable acids, with the exception of the compounds of formula (II) in which $Y_1$ represents CO, n represents 2, and $R_1$ and $R_2$ simultaneously represent hydrogen, compounds of formula (II) in which $Y_1$ represents CO, $X_1$ represents F, Cl, Br in the ortho, meta or para position or I in the ortho or meta position, compounds of formula (II) wherein $Y_1$ denotes $SO_2$ and $X_1$ denotes Cl in the para position and the compound of formula II wherein $Y_1$ denotes CO, $X_1$ is an iodine atom in a para position and $R_1$ and $R_2$ represent an ethyl group.

**8.** Pharmaceutical agent according to Claim 5, characterised in that it consists of N-(2-[ethylamino]-ethyl)-4-iodobenzamide and the hydrochloride thereof.

**9.** Pharmaceutical agent according to Claim 5, characterised in that it consists of N-(2-[dimethylamino]-ethyl)-4-iodobenzamide.

**10.** Pharmaceutical agent according to Claim 5, characterised in that it consists of N-(2-[methylamino]-ethyl)-4-iodobenzamide.

**11.** Pharmaceutical agent according to Claim 5, characterised in that it consists of N-(2- (diisopropylamino]-ethyl)-4-iodobenzamide.

**12.** Pharmaceutical agent according to Claim 5, characterised in that it consists of N-(2-[isopropylamino]-ethyl)-4-iodobenzamide.

**13.** Pharmaceutical agent according to claim 5, characterised in that it consists of N-(3-[dimethylamino]-propyl)-4-iodobenzamide.

**14.** Pharmaceutical agent according to Claim 5, characterised in that it consists of N-(3-[methylamino]-propyl)-4-iodobenzamide.

**15.** Pharmaceutical agent according to Claim 5, characterised in that it consists of N-(2-[diethylamino]-ethyl)-4-iodobenzamide.

**16.** Use of N-(2-[diethylamino]-ethyl)-2-iodobenzamide for the preparation of a radiopharmaceutical agent for diagnosing and treating malignant melanomas.

**17.** Use of N-(3-[diethylamino]-propyl)-4-iodobenzamide for the preparation of a radiapharmaceutical agent for diagnosing and treating malignant melanomas.

**18.** Use of N-(2-[ethylamino]-ethyl)-4-iodobenzamide or the hydrochloride thereof for the preparation of a radiopharmaceutical agent for diagnosing and treating malignant melanomas.

**19.** Use of N-(2-[dimethylamino]-ethyl)-4-iodobenzamide for the preparation of a radiopharmaceutical agent for diagnosing and treating malignant melanomas.

**20.** Use of N-(2-[methylamino]-ethyl)-4-iodobenzamide for the preparation of a radiopharmaceutical agent for diagnosing and treating malignant melanomas.

**21.** Use of N-(2-[diisopropylamino]-ethyl)-4-iodobenzamide for the preparation of a radiopharmaceutical agent for diagnosing and treating malignant melanomas.

**22.** Use of N-(2-[isopropylamino]-ethyl)-4-iodobenzamide for the preparation of a radiopharmaceutical agent for diagnosing and treating malignant melanomas.

**23.** Use of N-(3-[dimethylamino]-propyl)-4-iodobenzamide for the preparation of a radiopharmaceutical agent for diagnosing and treating malignant melanomas.

**24.** Use of N-(3-[methylamino]-propyl)-4-iodobenzamide for the preparation of a radiopharmaceutical agent for diagnosing and treating malignant melanomas.

**25.** Use of N-(2-[diethylamino]-ethyl)-4-iodobenzamide for the preparation of a radiopharmaceutical agent for diagnosing and treating malignant melanomas.

**26.** Use of N-(2-aminoethyl)-4-iodobenzamide for the preparation of a radiopharmaceutical agent for diagnosing and treating malignant melanomas.

**27.** Agents labelled with stable isotopes selected from $^{13}C$ and $^{19}F$ according to Claim 5 for use in nuclear magnetic resonance imaging.

## Patentansprüche

**1.** Verwendung einer Verbindung der allgemeinen Formel

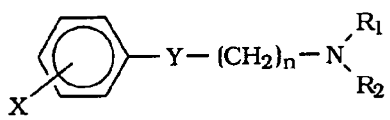

(I)

in der
X ein Wasserstoffatom oder ein Halogenatom,
Y eine Gruppe Z-CONH-, Z-O-, Z-S(O)-$_{n1}$, worin $n_1$ = 0, 1, 2 oder 3 darstellt, Z-$SO_2$-NH- oder Z-NHCO-, worin Z eine Bindung, -$CH_2$- oder

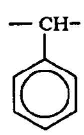

darstellt,
n eine ganze Zahl mit einem Wert von 2 bis 4 und
$R_1$ und $R_2$ jeweils ein Wasserstoffatom, eine $C_1$-$C_6$-Alkylgruppe oder eine $C_6$-$C_{12}$-Aryl- oder Aralkylgruppe bedeuten, wobei diese Verbindungen durch Radioisotope ausgewählt aus $^{123}I$, $^{125}I$, $^{131}I$, $^{75}Br$, $^{77}Br$, $^{18}F$ oder $^{11}C$ oder stabilen Isotopen ausgewählt aus $^{13}C$ und $^{19}F$, markiert sein können,
sowie von deren Additionssalzen mit pharmazeutisch annehmbaren Säuren zur Herstellung eines Mittels für die Diagnose und/oder die Behandlung von bösartigen Melanomen.

2. Verwendung nach Anspruch 1 einer Verbindung der Formel (I), worin X $^{123}I$, $^{125}I$, $^{131}I$, Y Z-CONH- oder Z-$SO_2$-NH-, worin Z eine Bindung, -$CH_2$- oder

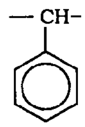

darstellt und
$R_1$ und $R_2$ jeweils ein Wasserstoffatom oder eine $C_1$-$C_6$-Alkylgruppe bedeuten.

3. Verwendung der Verbindung der Formel

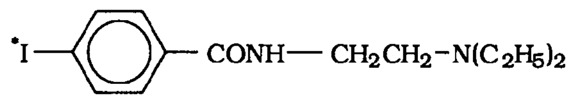

worin *I für $^{125}I$, $^{123}I$ oder $^{131}I$ steht.
zur Herstellung eines radiopharmazeutischen Mittels zur Diagnose und zur Behandlung von bösartigen Melanomen.

4. Verwendung einer Verbindung der allgemeinen Formel

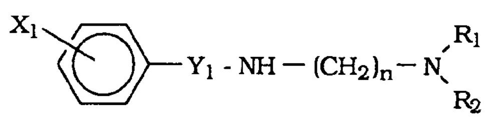
(II)

in der
$X_1$ ein Halogenatom oder eines seiner Radioisotope,
$Y_1$ eine CO- oder $SO_2$-Gruppe,
n eine ganze Zahl mit einem Wert von 2 bis 4 und
$R_1$ und $R_2$ jeweils ein Wasserstoffatom oder eine $C_1$-$C_6$-Alkylgruppe bedeuten, sowie von deren Additionssalzen mit pharmazeutisch annehmbaren Salzen zur Herstellung eines Mittels zur Diagnose und/oder zur Behandlung von bösartigen Melanomen.

5. Pharmazeutische Mittel entsprechend der allgemeinen Formel

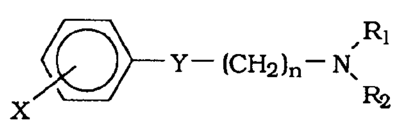
(I)

nach Anspruch 1, worin
X eine Wasserstoffatom oder ein Halogenatom,
Y eine Gruppe Z-CONH-, Z-O-, Z-S(O)-$_{n1}$, worin $n_1$ = 0, 1, 2 oder 3 darstellt, Z-$SO_2$-NO- oder Z-NHCO-, worin Z eine Bindung, -$CH_2$- oder

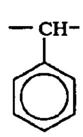

darstellt,
n eine ganze Zahl mit einem Wert von 2 bis 4 und

$R_1$ und $R_2$ jeweils ein Wasserstoffatom, eine $C_1$-$C_6$-Alkylgruppe oder eine $C_6$-$C_{12}$-Aryl- oder -Aralkylgruppe bedeuten,
welche Verbindungen durch Radioisotope ausgewählt aus $^{123}I$, $^{125}I$, $^{131}I$, $^{75}BR$, $^{77}Br$, $^{18}F$ oder $^{11}C$ oder stabilen Isotopen ausgewählt aus $^{13}C$ und $^{19}F$, markiert sein können,
sowie deren Additionssalze mit pharmazeutisch annehmbaren Säuren, mit Ausnahme der Verbindungen der Formel (I), worin Y Z-CONH-, worin Z eine Bindung darstellt, n 2 und $R_1$ und $R_2$ gleichzeitig ein Wasserstoffatom bedeuten. der Verbindungen der Formel (I), in der Y Z-CONH-, worin Z eine Bindung darstellt, und X F, Cl, Br in der ortho-, meta- oder para-Stellung oder I in der ortho- oder meta-Stellung bedeuten, der Verbindungen der Formel (I), worin Y Z-$SO_2$-NH-, worin Z eine Bindung darstellt, und X Cl in der para-Stellung bedeuten, und der Verbindung der Formel (I), in der Y Z-CO-NH-, worin Z eine Bindung darstellt, X ein Iodatom in der para-Stellung und $R_1$ und $R_2$ eine Ethylgruppe bedeuten.

6. Mittel nach Anspruch 5 der Formel (I), in der X $^{123}I$, $^{125}I$ oder $^{131}I$, Y Z-CONH- oder Z-$SO_2$-NH-, worin Z eine Bindung, -$CH_2$- oder

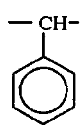

darstellt, und
$R_1$ und $R_2$ jeweils ein Wasserstoffatom oder eine $C_1$-$C_6$-Alkylgruppe bedeuten.

7. Mittel nach Anspruch 5 der allgemeinen Formel

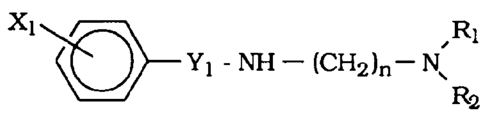

(II)

in der
$X_1$ ein Halogenatom oder eines seiner Radioisotope,
$Y_1$ eine CO- oder $SO_2$-Gruppe.
n eine ganze Zahl mit einem Wert von 2 bis 4 und
$R_1$ und $R_2$ jeweils ein Wasserstoffatom oder eine $C_1$-$C_6$-Alkylgruppe bedeuten, sowie deren Additionssalze mit pharmazeutisch annehmbaren Säuren, mit Ausnahme der Verbindungen der Formel (II), in der $Y_1$ CO, n 2 und $R_1$ und $R_2$ gleichzeitig Wasserstoff bedeuten, der Verbindungen der Formel (II), in der $Y_1$ CO, $X_1$ F, Cl oder Br in der ortho-, meta- oder para-Stellung oder I in der ortho- oder meta-Stellung bedeuten, der Verbindungen der Formel (II), in der $Y_1$ $SO_2$ und $X_1$ Cl in der para-Stellung bedeuten und der Verbindung der Formel (II). in der $Y_1$ CO, $X_1$ ein Iodatom in der para-Stellung und $R_1$ und $R_2$ eine Ethylgruppe bedeuten.

8. Pharmazeutisches Mittel nach Anspruch 5, **dadurch gekennzeichnet,** daß es aus N-(2-Ethylamino-ethyl)-4-iod-benzamid und dessen Hydrochlorid besteht.

9. Pharmazeutisches Mittel nach Anspruch 5, **dadurch gekennzeichnet**, daß es aus N-(2-Dimethylamino-ethyl)-4-iod-benzamid besteht.

10. Pharmazeutisches Mittel nach Anspruch 5, **dadurch gekennzeichnet**, daß es aus N-(2-Methylamino-ethyl)-4-iod-benzamid besteht.

11. Pharmazeutisches Mittel nach Anspruch 5, **dadurch gekennzeichnet**, daß es aus N-(2-Diisopropylamino-ethyl)-4-iod-benzamid besteht.

12. Pharmazeutisches Mittel nach Anspruch 5, **dadurch gekennzeichnet**, daß es aus N-(2-Isopropylamino-ethyl)-4-iod-benzamid besteht.

13. Pharmazeutisches Mittel nach Anspruch 5, **dadurch gekennzeichnet**, daß es aus N-(3-Dimethylamino-

propyl)-4-iod-benzamid besteht.

14. Pharmazeutisches Mittel nach Anspruch 5, **dadurch gekennzeichnet**, daß es aus N-(3-Methylamino-propyl) -4-iod-benzamid besteht.

15. Pharmazeutisches Mittel nach Anspruch 5, **dadurch gekennzeichnet,** daß es aus N-(2-Diethylamino-ethyl)-4-iod-benzolsulfonamid besteht.

16. Verwendung von N-(2-Diethylamino-ethyl)-2-iod-benzamid zur Herstellung eines radiopharmazeutischen Mittels zur Diagnose und zur Behandlung von bösartigen Melanomen.

17. Verwendung von N-(3-Diethylamino-propyl)-4-iod-benzamid zur Herstellung eines radiopharmazeutischen Mittels zur Diagnose und zur Behandlung von bösartigen Melanomen.

18. Verwendung von N-(2-Ethylamino-ethyl)-4-iod-benzamid oder seines Hydrochlorids zur Herstellung eines radiopharmazeutischen Mittels zur Diagnose und zur Behandlung von bösartigen Melanomen.

19. Verwendung von N-(2-Dimethylamino-ethyl)-4-iod-benzamid zur Herstellung eines radiopharmazeutischen Mittels zur Diagnose und zur Behandlung von bösartigen Melanomen.

20. Verwendung von N-(2-Methylamino-ethyl) -4-iod-benzamid zur Herstellung eines radiopharmazeutischen Mittels zur Diagnose und zur Behandlung von bösartigen Melanomen.

21. Verwendung von N-(2-Diisopropylamino-ethyl)-4-Iod-benzamid zur Herstellung eines radiopharmazeutischen Mittels zur Diagnose und zur Behandlung von bösartigen Melanomen.

22. Verwendung von N-(2-Isopropylamino-ethyl)-4-iod-benzamid zur Herstellung eines radiopharmazeutischen Mittels zur Diagnose und zur Behandlung von bösartigen Melanomen.

23. Verwendung von N-(3-Dimethylamino-propyl)-4-iod-benzamid zur Herstellung eines radiopharmazeutischen Mittels zur Diagnose und zur Behandlung von bösartigen Melanomen.

24. Verwendung von N-(3-Methylamino-propyl)-4-iod-benzamid zur Herstellung eines radiopharmazeutischen Mittels zur Diagnose und zur Behandlung von bösartigen Melanomen.

25. Verwendung von N-(2-Diethylamino-ethyl)-4-iod-benzolsulfonamid zur Herstellung eines radiopharmazeutischen Mittels zur Diagnose und zur Behandlung von bösartigen Melanomen.

26. Verwendung von (2-Aminoethyl) -4-iod-benzamid zur Herstellung eines radiopharmazeutischen Mittels zur Diagnose und zur Behandlung von bösartigen Melanomen.

27. Mit stabilen Isotopen ausgewählt aus $^{13}C$ und $^{19}F$ markierte Mittel nach Anspruch 5 zur Verwendung in der magnetischen Kernresonanzabbildungstechnik.